# EUROPEAN PATENT SPECIFICATION

(11) **EP 2 151 183 B1**
(45) Date of publication and mention of the grant of the patent: **06.06.2012**
(21) Application number: 09005713.4
(22) Date of filing: 23.04.2009
(51) Int. Cl.: A61B 1/005

(54) **Flexible tube for endoscope and its manufacturing process**
Biegbarer Schlauch für Endoskop und dessen Herstellungsverfahren
Tube flexible pour endoscope et son procédé de fabrication

(30) Priority: 07.08.2008 JP 2008204457
(43) Date of publication of application: 10.02.2010
(73) Proprietor: Olympus Medical Systems Corp., Tokyo 151-0072 (JP)
(72) Inventor: Ogura, Hitoshi, Hachioji-shi Tokyo 192-8512 (JP)
(74) Representative: von Hellfeld, Axel

(56) References cited:
- EP-A- 0 615 832
- US-A- 5 885 209
- US-A1- 2005 288 545

## Description

The present invention relates to a flexible tube, and specifically to an endoscopic flexible tube and a method for producing the same.

Endoscopes are reused, and must be washed and disinfected after every use. Therefore, the sheaths of endoscopic flexible tubes must be impermeable to body fluids, cleaning solvents, and antiseptic solutions, and must have elasticity and flexibility suitable for insertion into body cavities.

Conventionally, the sheath of an endoscopic flexible tube is composed of a mixture of a thermoplastic polyester elastomer (TPC) and a thermoplastic polyurethane elastomer (TPU), or a mixture of TPU and TPC containing soft polyvinyl chloride (PVC). Jpn. Pat. Appln. KOKAI Publication No. 11-56762 suggests a product composed of a thermoplastic fluorocarbon elastomer.

As described above, the insertability of endoscopes depends on the flexibility and elasticity. For example, the flexible tube disclosed in Jpn. Pat. Appln. KOKAI Publication No. 11-56762 has low elasticity and thus unsatisfactory insertability, because it is composed of a fluorocarbon material having a specific gravity of 1.89 g/cm³, which is 1.5 or more times heavier than the specific gravity of TPU (1.1 to 1.25 g/cm³) or TPC (1.17 to 1.25 g/cm³).

US Patent Application Publication US 2005/0288545 A1 discloses a flexible tube for an endoscope comprising a helical tube, a net tube put on the helical tube, an outer skin put on an outer peripheral surface of the tube, and a topcoat put on a surface of the outer skin. The outer skin and the topcoat are molded by substantially simultaneously laminating resin materials together.

European Patent Application Publication EP 0 615 832 A1 discloses a flexible non-porous tube for an endoscope comprising a tubular substrate of porous expanded polytetrafluoroethylene having a microstructure of nodes interconnected by fibrils and having void spaces between fibrils. A portion of the void spaces between fibrils are filled with silicone rubber. The tube has a non-porous luminal surface comprising polytetrafluoroethylene and silicone rubber.

In order to reduce the burdens on the subject and operator, the insertability of endoscopes must be improved. In order to achieve this, the endoscopic flexible tubes must have high elasticity while maintaining the existing flexibility. In addition, along with the diversification of sterilization methods, the endoscopic flexible tubes are required to have higher heat resistance.

The present invention has been accomplished in view of the above-described problems, and is intended to provide an endoscopic flexible tube having high elasticity while maintaining the existing flexibility, and a method for producing the same.

In order to achieve the objects, a first aspect of the present invention relates to an endoscopic flexible tube, including a spiral tube, a mesh tube covering the spiral tube, and a sheath coating the outer surface of the mesh tube, wherein the sheath is composed of a sheathing resin material containing closed cells.

The closed cells are formed with hollow microspheres.

The hollow microspheres are made of glass. The loading of the hollow microspheres is 45 parts by weight or less with respect to 100 parts by weight of the sheathing resin material.

A second aspect of the present invention relates to the endoscopic flexible tube, characterized in that the hollow microspheres have an average particle size of 5 to 135 µm.

A third aspect of the present invention relates to a method for producing an endoscopic flexible tube, preferably including covering a spiral tube with a mesh tube to form a flexible tube core, kneading a sheathing resin material together with hollow glass microspheres to make a sheathing material, and coating the outer surface of the flexible tube core with the sheathing material to obtain an endoscopic flexible tube, wherein the loading of the hollow microspheres is 45 parts by weight or less with respect to 100 parts by weight of the sheating resin material.

According to the endoscopic flexible tube and the method for producing the same according to the present invention, there is provided an endoscopic flexible tube having high elasticity while maintaining the existing flexibility.

The invention can be more fully understood from the following detailed description when taken in conjunction with the accompanying drawings, in which:
FIG. 1 is a perspective view showing the schematic structure of an endoscope 10 according to a first embodiment;
FIG. 2 is a schematic view of an endoscopic flexible tube 1 according to the first embodiment;
FIG. 3 is a cross-sectional view of the endoscopic flexible tube 1 according to the first embodiment;
FIG. 4 is an enlarged view of a sheath 6 according to the first embodiment; and
FIG. 5 shows schematic views of the sheath 6 in various forms.

Hereinafter, embodiments of the invention will be described with reference to the drawings. Incidentally, the embodiments described below are merely examples for explaining the structure of the invention in detail. Accordingly, the invention should not be restrictively interpreted based on the description of the following embodiments. The scope of the invention includes all embodiments including various modifications and improvements of the following embodiments within the scope of the invention as set forth in the claims.

### <First embodiment>

FIG. 1

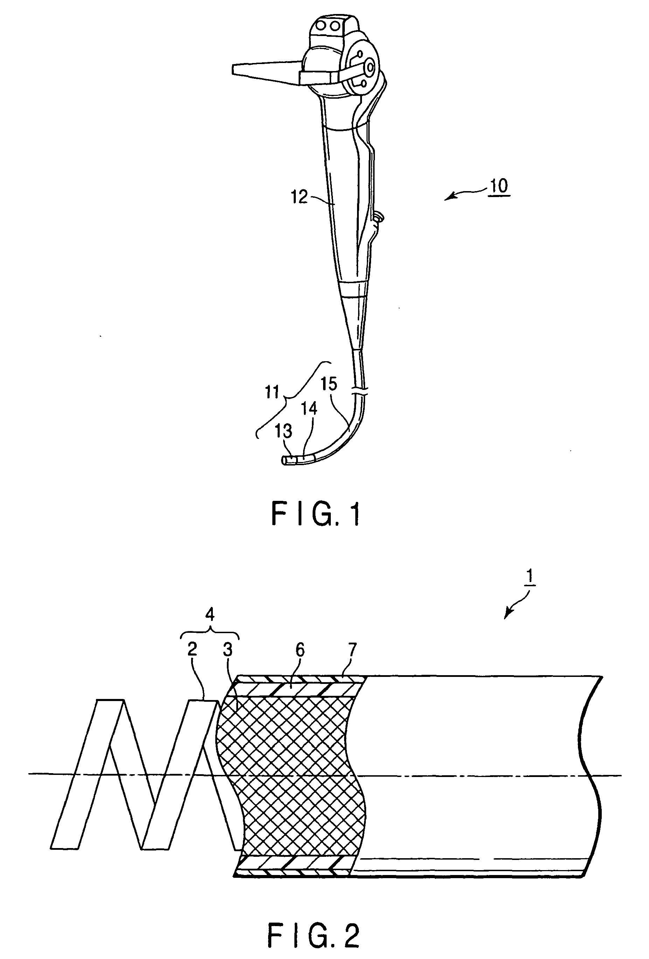

   FIG. 1 is a perspective view showing the schematic structure of an endoscope 10 according to a first embodiment. As shown in FIG. 1, the endoscope 10 includes an elongated flexible insert 11, and an operating unit 12 provided at the base of the insert 11. The insert 11 is composed of a toughened tip 13, a bending section 14 connected to the tip 13, and a flexible tube 15 connected to the bending section 14. The bending section 14 can be bent to a desired angle by remote control with the operating unit 12. The flexible tube 15 is a hose for inserting the tip 13 deeply into, for example, the body cavity such as the duodenum, small intestine, or large intestine.
FIGS. 2 and 3

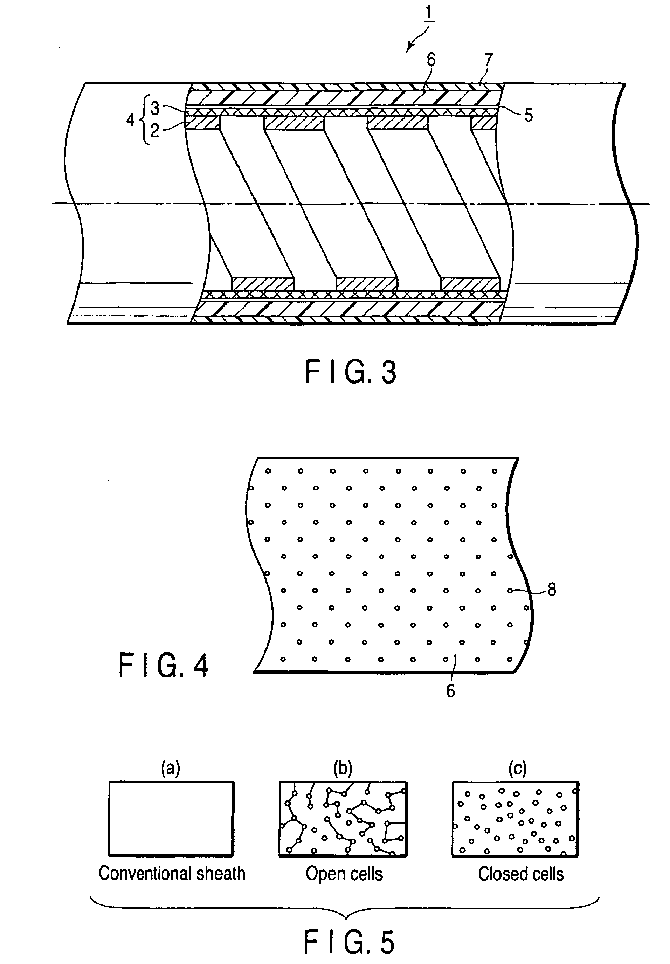

   FIGS. 2 and 3 are a schematic view and a cross-sectional view of the endoscopic flexible tube 1 according to the first embodiment, respectively.

As shown in FIGS. 2 and 3, the endoscopic flexible tube (hereinafter referred to as "flexible tube") 1 is composed of a flexible tube core 4, and a sheath 6 coating the outer surface of the flexible tube core 4. The flexible tube core 4 is composed of a spiral tube 2, and a mesh tube 3 coating the outer surface of the spiral tube 2.

The spiral tube 2 is a spirally wound elastic sheet. The elastic sheet may be made of stainless steel or a copper alloy. The mesh tube 3 is woven from a plurality of metallic or nonmetallic threads. The threads may be made of stainless steel or a synthetic resin. In order to improve the adhesion with the sheathing resin, the mesh tube 3 may be woven from a mixture of metallic and nonmetallic threads.

The sheath 6 coating the outer surface of the flexible tube core 4 may be composed of any known thermoplastic elastomer such as a thermoplastic polyester elastomer (TPC), a thermoplastic polyurethane elastomer (TPU), a thermoplastic fluorocarbon elastomer, a thermoplastic olefin elastomer, or a thermoplastic styrene elastomer. The sheath 6 may be composed of a crosslinked rubber.

The outer surface of the sheath 6 may be further coated with a top coat 7. The top coat 7 is made of a thin material with excellent chemical resistance and smoothness on the body wall of a patient. The top coat 7 may be made of a urethane-based or fluorocarbon resin. In the endoscopic flexible tube 1 of the present invention, the flexible tube core 4 is coated with a sheath containing closed cells. Therefore, the endoscopic flexible tube 1 having the sheath 6 as the outermost layer provides high elasticity and impermeability, regardless of the presence or absence of the top coat 7. Therefore, the step of forming the top coat 7 may be omitted from the manufacturing process of the endoscopic flexible tube 1, thereby achieving the scale-down of the manufacturing apparatus, labor savings in procurement and preparation of raw materials, and reduction of the manufacturing cost. In this case, the weight and diameter of the endoscopic flexible tube 1 are further reduced.

Alternatively, as shown in FIG. 3, a layer of a coupling agent 5 may be provided on the outer surface of the flexible tube core 4, the layer being closely coated with the sheath 6 composed of a thermoplastic elastomer. The coupling agent 5 may be a silane coupling agent. Other examples of the coupling agent include titanate-based, aluminum-based, and zirconium-based coupling agents. The coupling agent may contain a pigment for facilitating the distinction of whether the coupling agent is applied.
FIGS. 4 and 5
FIG. 4 is an enlarged view of the sheath 6 according to the first embodiment. The sheath 6 contains a plurality of closed cells 8. FIG. 5 shows schematic views of the sheath 6 in various forms.

As shown in FIG. 4, a plurality of closed cells 8 are dispersed in the sheath 6 of the flexible tube according to the present invention. The closed cells 8 dispersed in the sheath 6 reduce the specific gravity of the sheath 6, thereby achieving the high elasticity of the flexible tube 1.

FIG. 5 shows different sheath structures. The conventional sheath 6 is composed of a uniform resin material (FIG. 5A). The conventional sheath 6 has a high specific gravity because it contains no cavity, and thus cannot provide high elasticity. On the other hand, a sheath produced with, for example, a foaming agent contains open cells (FIG. 5B). The sheath 6 containing open cells is not completely impermeable to water. Therefore, the sheath 6 allows penetration of pathogens thereinto, which hinders sufficient sterilization. Even if cavities are formed using a foaming agent, the cavities disappear over time, which results in the thinning of the sheath and the variation of the outside diameter. On the other hand, the sheath 6 according to the first embodiment contains closed cells formed with hollow microspheres or a porous material (FIG. 5C). Since the sheath 6 containing closed cells has a low specific gravity, it provides high elasticity. In addition, since the cavities are independent, the sheath 6 has water impermeability equivalent to the conventional sheath, and does not allow penetration of pathogens thereinto. Therefore, the sheath 6 will not suffer from contamination, and thus is reusable after any treatment such as washing, disinfection, or low-temperature plasma sterilization. Since the cavities will not be disappeared by heating, the sheath 6 may be sterilized with high-pressure steam in an autoclave thereby more securely preventing infection, and the ease of insertion is maintained in the long term.

The closed cells contained in the sheath 6 according to the present invention may be formed through the addition of hollow microspheres. The hollow microspheres are fine hollow bodies having a cavity inside. The hollow microspheres have a high strength and high heat resistance, and will not be collapsed or melted during molding of the resin material composing the sheath 6. Therefore, the hollow microspheres provide stable closed cells within the sheath 6. The hollow microspheres may be made of glass.

The average particle size of the hollow microspheres is from 1 to 1000 µm, preferably from 2 to 500 µm, more preferably from 3 to 200 µm, and most preferably from 5 to 135 µm. Hollow microspheres having a smaller average particle size are more finely dispersed to increase the elongation of the sheathing resin material.

The density of the hollow microspheres depends on the density of the sheathing resin material. The specific gravity of the sheath 6 containing hollow microspheres must be lower than that composed of a resin material alone and containing no microsphere. In usual cases, the true density of the hollow microspheres is 1.8 g/cm³ or less, preferably 1.1 g/cm³ or less, the apparent density is 1.5 g/cm³ or less, preferably 0.8 g/cm³ or less, and the bulk density is 1.0 g/cm³ or less, preferably 0.5 g/cm³ or less. The porosity of the hollow microspheres is from 10 to 99%, preferably from 50 to 98%, and more preferably from 70% to 95%. Hollow microspheres having a smaller true density more reduce the specific gravity of the sheathing material, and are highly effective in a small dose.

The hollow microspheres are thermally stable, and specifically have a softening temperature of 300°C or higher, preferably 500°C or higher, and more preferably 550°C or higher. The hollow microspheres are highly resistant to pressure, and specifically has a pressure capacity of 0.1 MPa or more, preferably 1.0 MPa or more, and more preferably 1.7 MPa or more.

The hollow glass microspheres are composed of, for example, SiO₂, B₂O₃, Na₂O, CaO, Al₂O₃, Fe₂O₃, K₂O, MgO, ZnO, TiO₂, or P₂O₅. The hollow glass microspheres may be made of soda lime borosilicate glass. High-quality commercial hollow glass microspheres are widely available, and examples thereof include GLASS BUBBLES (Sumitomo 3M Ltd.), Q-CELL (Potters Industries Inc.), and E-SPHERES (Taiheiyo Cement Corporation). The flexible tube 1 according to the present invention may be produced with these microspheres. Hollow resin microspheres are composed of, for example, a thermosetting resin such as an epoxy or phenol resin.

These hollow microspheres may be used alone or in combination.

Hollow microspheres are usually classified into fillers. If the loading of the hollow microspheres is too high, the sheathing material may be too hard or brittle. Accordingly, the loading of the hollow microspheres is 45 parts by weight or less with respect to 100 parts by weight of the sheathing resin material, in order to reduce the specific gravity of the sheath 6 to increase its elasticity while preventing embrittlement.

Alternatively, a porous material may be used in place of the hollow microspheres to obtain the same structure; which alternative is not falling within the scope of the claims .

Other fillers may be added as necessary. Specific examples of the fillers include inorganic fillers such as carbon black, silica, barium sulfate, titanium oxide, aluminum oxide, calcium carbonate, calcium silicate, magnesium silicate, and aluminium silicate, and organic fillers such as polytetraluoroethylene resins, polyethylene resins, polypropylene resins, phenolic resins, polyimide resins, melamine resins, and silicone resins. These fillers may be used in combination.

In addition, fibers may be added as desired. Specific examples of the fibers include inorganic fibers such as glass fibers, alumina fibers, and rook wool, and organic fibers such as cotton, wool, silk, hemp, nylon fiber, aramid fibers, vinylon fibers, polyester fibers, rayon fibers, acetate fibers, phenolformaldehyde fibers, polyphenylenesulfide fibers, acrylic fibers, polyvinyl chloride fibers, polyvinylidene chloride fibers, polyurethane fibers, and tetrafluoroethylene fibers. These fibers may be used in combination.

In addition, other additives such as lubricants, stabilizers, weathering stabilizers, ultraviolet absorbers, and antistatic agents may be added without impairing the advantages of the invention.

The method for producing the endoscopic flexible tube 1 according to the first embodiment will be described below.

First, a spiral tube 2 composed of a spirally wound elastic sheet, such as a stainless steel, is covered by a mesh tube 3 woven from, for example, synthetic resin threads.

Next, a layer of a coupling agent 5 is provided on the outer surface of the mesh tube 3, and the top of the layer is closely coated with a sheath 6. The layer of the coupling agent 5 is not essential, and the sheath 6 may be provided directly on the mesh tube 3. In this case, it is preferred that the mesh tube 3 is preliminarily coated with a highly penetrative adhesive such as a urethane adhesive.

The formation of the sheath 6 according to the present invention may use various conventional methods. Usually, melt kneading with, for example, a kneader, a Banbury mixer, or a continuous kneading extruder is used. The kneading temperature is not particularly limited, insofar as the components are uniformly dispersed, and the temperature is not higher than the decomposition temperature of the materials. Specifically, the sheath 6 is formed by mixing, for example, a thermoplastic polyester elastomer (TPC) with a specified amount of hollow glass microspheres, kneading the mixture with a continuous kneading extruder, and the kneaded product is extruded to form the sheath 6.

Finally, a resin with excellent chemical resistance and smoothness, such as a urethane or fluorocarbon resin, is deposited on the sheath 6 to a predetermined thickness by dipping or extrusion, thereby forming the top coat 7. The formation of the top coat 7 may be carried out concurrently with coating with the sheath 6. Since the sheath 6 according to the first embodiment contains closed cells, it has a low specific gravity and high elasticity, as well as excellent impermeability and chemical resistance. Therefore, the top coat 7 is not essential for the flexible tube 1 including the sheath 6 according to the first embodiment.

### [Example]

The present invention will be further described with reference to the following examples, but the present invention is not limited to the examples.

### 1. Making of endoscopic flexible tube

The elastomers and fillers (hollow glass microspheres) listed in Table 1 were used to make the sheaths 6, with which endoscopic flexible tubes were produced. The elastomers were polyester, polyurethane, polyolefin, and fluorocarbon elastomers, and the fillers were GLASS BUBBLES K1 and A20 (Sumitomo 3M Ltd.), Q-CELL 7014 (Potters Industries, Inc.), and E-SPHERES (Taiheiyo Cement Corporation).

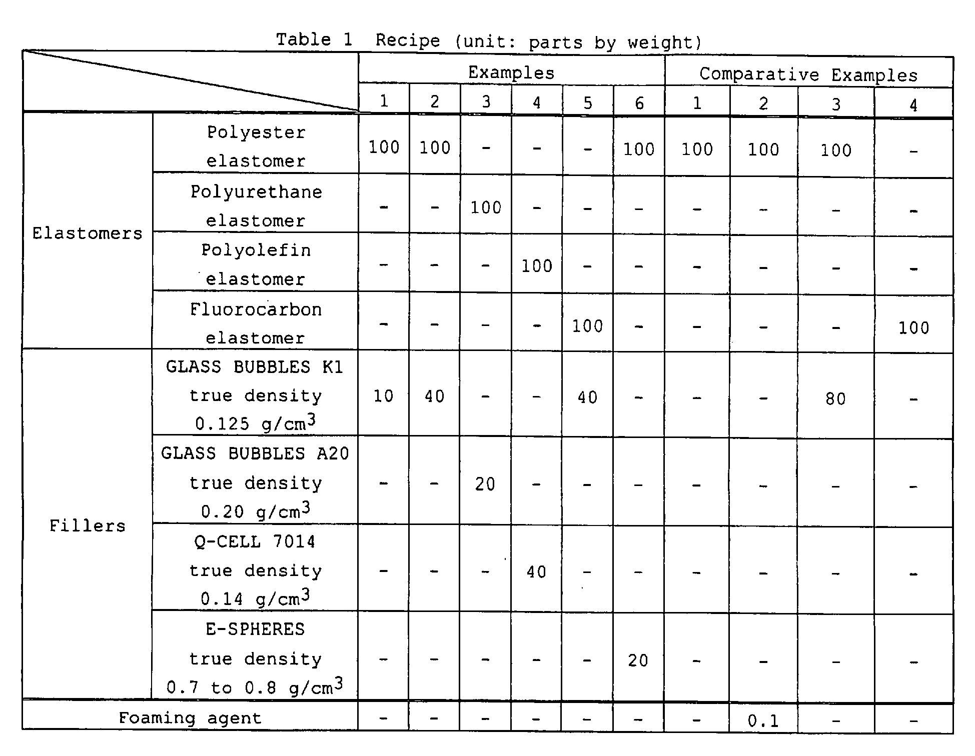

### [Example 1]

10 parts by weight of hollow glass microspheres (GLASS BUBBLES K1, true density: 0.125 g/cm³) were mixed with 100 parts by weight of a thermoplastic polyester elastomer (TPC), and the mixture was kneaded with a continuous kneading extruder to obtain a sheathing material. A core composed of a spiral tube covered by a mesh tube was coated on its outer surface with the sheathing material, thereby producing an endoscopic flexible tube.

### [Example 2]

40 parts by weight of hollow glass microspheres (GLASS BUBBLES K1, true density: 0.125 g/cm³) were mixed with 100 parts by weight of a thermoplastic polyester elastomer (TPC), and the mixture was kneaded with a continuous kneading extruder to obtain a sheathing material. A core composed of a spiral tube covered by a mesh tube was coated on its outer surface with the sheathing material, thereby producing an endoscopic flexible tube.

### [Example 3]

20 parts by weight of hollow glass microspheres (GLASS BUBBLES A20, true density: 0.20 g/cm³) were mixed with 100 parts by weight of a thermoplastic polyurethane elastomer (TPU), and the mixture was kneaded with a continuous kneading extruder to obtain a sheathing material. A core composed of a spiral tube covered by a mesh tube was coated on its outer surface with the sheathing material, thereby producing an endoscopic flexible tube.

### [Example 4]

40 parts by weight of hollow glass microspheres (Q-CELL 7014, true density: 0.14 g/cm³) were mixed with 100 parts by weight of a thermoplastic polyolefine elastomer, and the mixture was kneaded with a continuous kneading extruder to obtain a sheathing material. A core composed of a spiral tube covered by a mesh tube was coated on its outer surface with the sheathing material, thereby producing an endoscopic flexible tube.

### [Example 5]

40 parts by weight of hollow glass microspheres (GLASS BUBBLES K1, true density: 0.125 g/cm³) were mixed with 100 parts by weight of a thermoplastic fluorocarbon elastomer, and the mixture was kneaded with a continuous kneading extruder to obtain a sheathing material. A core composed of a spiral tube covered by a mesh tube was coated on its outer surface with the sheathing material, thereby producing an endoscopic flexible tube.

### [Example 6]

20 parts by weight of hollow glass microspheres (E-SPHERES, true density: 0.7 to 0.8 g/cm³) were mixed with 100 parts by weight of a thermoplastic polyester elastomer (TPC), and the mixture was kneaded with a continuous kneading extruder to obtain a sheathing material. A core composed of a spiral tube covered by a mesh tube was coated on its outer surface with the sheathing material, thereby producing an endoscopic flexible tube.

### [Comparative Example 1]

A core composed of a spiral tube covered by a mesh tube was coated on its outer surface with a sheathing material composed of a thermoplastic polyester elastomer (TPC) alone and containing no hollow glass microsphere, thereby producing an endoscopic flexible tube.

### [Comparative Example 2]

0.1 part by weight of a foaming agent (VINYFOR AC#3, Eiwa Chemical Ind. Co., Ltd.) was added to 100 parts by weight of a thermoplastic polyester elastomer (TPC) containing no hollow glass microsphere, thus obtaining a sheathing material containing open cells. A core composed of a spiral tube covered by a mesh tube was coated on its outer surface with the sheathing material, thereby producing an endoscopic flexible tube.

### [Comparative Example 3]

In order to examine the influence of an excess amount of hollow glass microspheres, 80 parts by weight of hollow glass microspheres (GLASS BUBBLES K1, true density: 0.125 g/cm³) were mixed with 100 parts by weight of a thermoplastic polyester elastomer, and the mixture was kneaded with a continuous kneading extruder to obtain a sheathing material. A core composed of a spiral tube covered by a mesh tube was coated on its outer surface with the sheathing material, thereby producing an endoscopic flexible tube.

### [Comparative Example 4]

In order to examine the influence of an elastomer with a high specific gravity, a core composed of a spiral tube covered by a mesh tube was coated on its outer surface with a sheathing material composed of a thermoplastic fluorocarbon elastomer alone and containing no hollow glass microsphere, thereby producing an endoscopic flexible tube.

### 2. Test result

**Table 2**

| | Elasticity | Water impermeability | Flexibility |
|---|---|---|---|
| Example 1 | Ⓞ | ○ | ○ |
| Example 2 | Ⓞ | ○ | ○ |
| Example 3 | Ⓞ | ○ | ○ |
| Example 4 | Ⓞ | ○ | ○ |
| Example 5 | Ⓞ | ○ | ○ |
| Example 6 | ○ | ○ | ○ |
| Comparative Example 1 | Δ | ○ | ○ |
| Comparative Example 2 | Ⓞ | × | ○ |
| Comparative Example 3 | Ⓞ | ○ | × |
| Comparative Example 4 | × | ○ | ○ |

| | | | |
|---|---|---|---|
| The symbols represent the followings. Ⓞ: Markedly good, ○: Good, Δ: Acceptable, ×: Unacceptable | | | |

As is evident from Table 2, the flexible tubes produced in Examples 1 to 6 received good ratings for the elasticity, water impermeability, and flexibility. On the other hand, the flexible tubes produced in Comparative Examples 1 to 4 received an unsatisfactory rating for the elasticity, water impermeability, or flexibility.

## Claims

1. An endoscopic flexible tube comprising:
a spiral tube (2);
a mesh tube (3) which covers the spiral tube (2); and
a sheath (6) which coats an outer surface of the mesh tube (3), wherein
the sheath (6) is composed of a sheathing resin material containing closed cells formed with hollow microspheres,
**characterized in that** the hollow microspheres are made of glass, and that loading of the hollow microspheres is 45 parts by weight or less with respect to 100 parts by weight of the sheathing resin material.

2. The endoscopic flexible tube according to claim 1, **characterized in that** the hollow microspheres have an average particle size of 5 to 135 [micro]m.

3. A method for producing an endoscopic flexible tube, comprising:
covering a spiral tube (2) with a mesh tube (3) to form a flexible tube core (4);
kneading a sheathing resin material together with hollow glass microspheres to make a sheathing material; and
coating an outer surface of the flexible tube core with the sheathing material to obtain an endoscopic flexible tube, **characterized in that** loading of the hollow glass microspheres is 45 parts by weight or less with respect to 100 parts by weight of the sheathing resin material.

## Patentansprüche

1. Biegbarer Schlauch für ein Endoskop mit:
einem Spiralschlauch (2);
einem Geflechtschlauch (3), der den Spiralschlauch (2) bedeckt; und
einer Umhüllung (6), die eine Außenfläche des Geflechtschlauchs (3) überzieht, wobei
die Umhüllung (6) aus einem Umhüllungs-Harzmaterial zusammengesetzt ist, das mit hohlen Mikrokugeln gebildete geschlossene Zellen enthält,
**dadurch gekennzeichnet, dass** die hohlen Mikrokugeln aus Glas hergestellt sind, und dass ein Gehalt an hohlen Mikrokugeln 45 Gewichtsanteile oder weniger in Bezug auf 100 Gewichtsanteile des Umhüllungs-Harzmaterials beträgt.

2. Biegbarer Schlauch für ein Endoskop nach Anspruch 1, **dadurch gekennzeichnet, dass** die hohlen Mikrokugeln eine durchschnittliche Partikelgröße von 5 bis 135 [micro]m haben.

3. Verfahren zur Herstellung eines biegbaren Schlauchs für ein Endoskop, umfassend:
Bedecken eines Spiralschlauchs (2) mit einem Geflechtschlauch (3), um einen flexiblen Schlauchkern (4) zu bilden;
Verkneten eines Umhüllungs-Harzmaterials zusammen mit hohlen Glas-Mikrokugeln, um ein Umhüllungsmaterial herzustellen; und
Beschichten einer Außenfläche des flexiblen Schlauchkerns mit dem Umhüllungsmaterial, um einen biegbaren Schlauch für ein Endoskop zu erhalten, **dadurch gekennzeichnet, dass** ein Gehalt der hohlen Glas-Mikrokugeln 45 Gewichtsanteile oder weniger in Bezug auf 100 Gewichtsanteile des Umhüllungs-Harzmaterials beträgt.

## Revendications

1. Tube flexible endoscopique comprenant :
un tube spiralé (2) ;
un tube grillagé (3) qui couvre le tube spiralé (2) ; et
une gaine (6) qui revêt une surface extérieure du tube grillagé (3), dans lequel la gaine (6) est composée d'un matériau de gainage en résine contenant des cellules fermées formées avec des microsphères creuses,
**caractérisé en ce que** les microsphères creuses sont réalisées en verre, et **en ce que** la charge de microsphères creuses est de 45 parts en poids ou moins par rapport à 100 parts en poids du matériau de gainage en résine.

2. Tube flexible endoscopique selon la revendication 1, **caractérisé en ce que** les microsphères creuses ont une taille de particules moyenne de 5 à 135 µm.

3. Procédé pour produire un tube flexible endoscopique, comprenant les étapes consistant à :
couvrir un tube spiralé (2) avec un tube grillagé (3) pour former un coeur de tube flexible (4) ;
malaxer un matériau de gainage en résine ensemble avec des microsphères en verre creuses pour produire un matériau de gainage ; et
revêtir une surface extérieure du coeur de tube flexible avec le matériau de gainage pour obtenir un tube flexible endoscopique, **caractérisé en ce que** la charge des microsphères en verre creuses est de 45 parts en poids ou moins par rapport à 100 parts en poids du matériau de gainage en résine.
